# EUROPEAN PATENT APPLICATION

(11) **EP 2 332 585 A1**
(43) Date of publication of application: **15.06.2011**
(21) Application number: 10179859.3
(22) Date of filing: 26.11.2004
(51) Int. Cl.: A61L 26/00, A61K 9/70

(54) **An adhesive patch**

(30) Priority: 28.11.2003 DK 200301761; 10.12.2003 US 528183 P
(62) Divisional of application: 04797482.9
(71) Applicant: Coloplast A/S, 3050 Humlebaek (DK)
(72) Inventor: Hansen, Grazyna, 2000, Frederiksberg C (DK); Nielsen, Anders Christian, 2880, Bagsvaerd (DK)

(57) **Abstract**

A patch for covering a portion of the anatomical surface of a living being, said patch being able to adhere to the skin or mucosa, and/or a wound, said patch comprising a backing layer and a layer of a skin-friendly adhesive for adhering to the skin or mucosa, said adhesive comprises hydrocolloid particles, wherein at least along the periphery of the patch the thickness of the adhesive layer is 20-300 µm and the vapour permeability of the patch is 200-1000 g/m².

## Description

### BACKGROUND OF THE INVENTION

### 1. Field of the Invention

The present invention relates to adhesive patches, in particular patches for covering, healing and treating a portion of the anatomical surface of a human being and a method for preparing such patches.

### 2. Description of the Related Art

Conventionally, dressings or patches for the treatment or prevention of wounds or pressure sores or even unbroken skin comprise a backing layer and an adhesive layer. The adhesive layer is often bevelled at the edge portions in order to have a smooth appearance, and avoid wrinkling and focusing of stresses in the dressing often causing slipping of the adhesive and unintended detachment of the dressing. The central portion of the dressing may be quite thick in order to provide a cushioning effect and/or an absorbent effect. However, a thick dressing may be less flexible and less capable of following the movements of the skin, as well as it appears very visible at the skin.

It is often desired to have a dressing or patch, which is very discreet or even "invisible" when applied to the skin. This may be the case when the patch is worn in the face or on other exposed areas. The patch may be used for covering scratches, wounds, acne, scars or discoloured skin and may have to purposes: To camouflage irregularities at the skin and/or to treat the skin site, e.g. a wound or acne. Different product for these purposes is known. These are usually in the form of adhesive patches or dots to be applied on the desired body part. Most of them are rather thick and thus rather unflexible and often quite visible. Some may contain medicaments for topical delivery.

Us Patent No. 5,785,978 discloses an adhesive patch comprising active ingredients. The patch comprises an occlusive backing film and an adhesive layer. The shape of the patch is adapted to the body part on which they are applied. The patch is occlusive and thus not breathable and does not comprise any absorbent elements and may thus give rise to unwanted humidity under the patch resulting in irritation of the skin and reduced wear time.

Aside from camouflaging the treated site of the skin, it may, especially in the treatment of Herpes, pebbles or eczema, be desired to provide the site with an antimicrobial barrier rendering the site more hygienic and decrease the risk of contamination.

Treatment of Herpes may be exceptionally difficult by the use of adhesive patches due to the fact that they have to adhere partly or fully to the lips and are exposed to large movements/stretching, friction and high humidity, all factors that may reduce wear time substantially, as well as it may be desired that the patch is as discrete as possible.

None of the known products address the problems associated with the problem of producing an adhesive patch being capable of staying in place and blending into the skin in such a way that it may appear almost invisible.

It has now been found that the patch of the present invention fulfils the above-mentioned demands by providing an almost invisible patch with long wear time.

### SUMMARY OF THE INVENTION

The invention relates to an adhesive patch for covering a portion of the anatomical surface of a human being, said patch being able to adhere to the skin or mucosa, and/or a wound, said patch comprising a backing layer and a layer of a skin-friendly adhesive for adhering to the skin or mucosa, said adhesive comprises hydrocolloid particles.

One object of the invention is to provide a non-occlusive adhesive patch, i.e. one that will enable moisture on the surface of the skin to evaporate through the patch so as to prevent the undesired accumulation of moisture, which, if occurred, could cause the patch to detach or even facilitate the growth of bacteria beneath the patch.

Another object of the invention is to provide a lighter, more flexible and less obtrusive patch while still providing the excellent wear time and healing properties.

Still another object of the present invention is to provide a patch for application in the face, especially the lip region.

Yet another object of the invention is to provide an adhesive patch being ultrathin or having ultra thin portions and capable of blending into the skin, rendering it extremely discreet for the user.

### Detailed Description of the Present Invention

The invention relates to an adhesive patch for covering a portion of the anatomical surface of a living being, said patch being able to adhere to the skin or mucosa, and/or a wound, said patch comprising a backing layer and a layer of a skin-friendly adhesive for adhering to the skin or mucosa, said adhesive comprises hydrocolloid particles, wherein at least along the periphery of the patch the thickness of the adhesive layer is 20-300 µm and the vapour permeability of the patch is 200-2000 g/m² and the absorption of the patch is 40-600 g/m²/6h.

The adhering surface preferably comprises hydrocolloid particles, the thickness of the adhesive being in the range of 25-300 µm, such as 30-200 µm, such as 25-150 µm, such as 30-100 µm, and the vapour permeability of the dressing sheet being 200-6000 g/m², such as 200-2000 g/m², such as 200-1000 g/m², such as 300-800 g/m², such as 400-700 g/m², such as 450-650 g/m² measures over 24 hours. It has been found that a dressing sheet with such thickness and vapour permeability provides a non-occlusive adhesive dressing sheet, i.e. one that enables moisture on, e.g., a skin surface to evaporate through the dressing sheet, so as to prevent undesired accumulation of moisture which could cause the dressing sheet to loose its adhering contact to the skin or promote bacterial growth between the sheet and the skin. Moreover, the low thickness of the dressing sheet results in a discrete appearance once applied to the application site.

The patch may preferably have an absorption of 50-400 g/m²/6h, more preferred 60-300 g/m²/6h and most preferred 70-250 g/m²/6h. The absorption is determined by immersing the patch in 0,9 M saline water at 37°C for 6 hours and then measuring the water uptake.

The presence of hydrocolloid in the adhesive provides a good environment for moist wound healing as well as for other skin conditions. By incorporating a limited amount of hydrocolloid in the adhesive, the patch is able to handle moisture in most conditions.

The present invention discloses a patch with a unique combination of a limited absorption combined with a high permeability. This combination provides optimal conditions for moist wound healing and long wear time when applied to skin or mucosa (e.g. lips).

Patches with very high permeability are known in the market, examples are SafeTac from Mölnlycke and Tielle from Johnson & Johnson. But these patches are not capable of providing moist wound healing. Products with a high absorption are also known. Examples of such are traditional hydrocolloid dressings. But these dressings may have a very low permeability until they are fully satisfied with moisture and then the permeability will rise, but due to the gelling of the adhesive the adhesive tack of the dressing will be reduced, and the dressing may delaminate from the skin. Furthermore, these dressings are rather thick and unflexible and thus unsuitable for treatment of e.g. Herpes.

The patch of the present invention may have a wear time on skin of at least 12 hours, more preferred at least 24 hours, even more preferred at least 36 hours and most preferred at least 48 hours. When applied to the mucosa and other places where the patch is exposed to high humidity and friction, such as in the lip region, the wear time may naturally be shorter. For application to the lip region, the wear time is preferably at least 2 hours, more preferred at least 3 hours and most preferred at least 4 hours.

It has surprisingly been shown that by using the patch of the present invention a thin but yet absorbing dressing may be used is cases where a thicker hydrocolloid dressing would traditionally have been chosen. Due to the high permeability of the patch of the present invention the patch may not need to be able to store large amounts of exudates/moisture, but due to evaporation the overall moisture handling capacity will be high, hence the flux of moisture through the patch is high.

The thickness of the dressing is often chosen from the amount of exudates expected from the wound, i.e. a thick dressing for a highly exuding wound. The idea is the thicker dressing, the more hydrocolloid particles and the higher absorption. However, a thick dressing may have a lower permeability. By using a thin patch with a high permeability, and due to the thickness, restricted amount of hydrocolloid particles, the moisture will be absorbed by the particles, thus not macerating the wound, and then the moisture will be released again through the top layer during evaporation. In this way the patch will be able to handle much higher amounts of moisture than the absorbency of the hydrocolloid particles.

Very thin dressings or patches are usually prepared with non-absorbent adhesives, such as polyacrylates. The presence of hydrocolloid particles, however, provides a moist wound healing environment by absorbing moisture, thus leaving the wound or skin neither too dry nor too wet. The absence of absorbent particles may give rise to maceration of the skin or drying out of a wound.

Though the term adhesive is used herein it is understood that the term may cover any substance having adherent properties, such as adhesives, silicone or rubbery substances, petrolatum or the like, and hydrocolloid adhesives. The adhesive may be a pressure sensitive adhesive of any suitable kind known per se.

The thickness of the adhesive layer of the patch of the present invention may be substantially constant over the surface or the patch may have a thicker portion at the centre of the patch, surrounded by a thinner periphery, i.e. a bevelled edge. It has surprisingly been shown that a better performance for the patch is achieved by having a thin edge portion. The thin periphery or the patch decreases the risk of rolling-up of the edge portion. The rolling up of the edge portion may lead to reduced wear time and is undesired. Furthermore, the thin edge portion is less exposed to water coming from outside, and renders it possible to obtain an extreme water-block.

It is preferred that the thickness of the adhesive layer is 20-200 µm, more preferred 25-150 µm and most preferred 30-100 µm and even most preferred 50-80 µm.

It may be preferred that the patch has a substantially uniform thickness. Due to the low thickness, bevelling may not be necessary in order to ensure good tack and reduce rolling up of the edge portions.

In one embodiment of the invention the patch is 100-200 µm thick. The obtained patch is thus thick enough to be handled without folding or wrinkling but at the same time remarkably thinner than traditional hydrocolloid dressings. Due to the permeability, this patch may be suitable for use on scratches and wounds, which normally would be treated with a thicker patch. Or it may suitable for such minor wounds or skin damages, where the high moisture capacity of a traditional thick hydrocolloid not is necessary. The patch of the present invention easily follows the movements of the skin and furthermore, it hardly takes up any place, which may be important when worn on the foot or toes being placed in a snug shoe.

A thin layer of adhesive is desired, as this will reduce the overall thickness of the patch. The thinner the dressing or patch is, the more flexible and more capable of following the movements of the body it is and the more discrete it appears.

The dressing or patch is especially suitable for use in the face or other visible or exposed areas of the body and it may therefore be desired that the patch blends into the skin and appears almost invisible.

Due to the reduced thickness of the patch it is possible to produce patches for scratches, wounds and for reducing scarring especially for exposed and/or protruding body parts. The patch may work like a second skin and easily follow the movements of the underlying skin.

The surface area of the dressing sheet may e.g. be 5-25 cm², such as 10-20 cm², or smaller, such as less than 5 cm², such as at most 4 cm², such as at most 2 cm², such as in the range of 1-2 cm², or smaller, such as 0.08 - 1 cm², such as 0.1 - 0.8 cm², such as 0.12 - 5 cm². For facial application, e.g. of a thin film patch, the surface area is usually less than 5 cm².

The vapour permeability of the patch is preferably 300-1100 g/m², more preferred 400-850 g/m², and most preferred 450-750 g/m².

The patch may have an optimal balance between a limited absorption and an appropriate permeability. Together these properties provides a patch with excellent moisture handling qualities compared to common thin dressings which may have a thicker layer of adhesive and/or a non-absorbing adhesive.

In one embodiment of the invention the patch may have bevelled edges. The bevelling may provide a smoother transition between the patch and the skin, rendering the patch more invisible.

The outer periphery of the patch is preferably bevelled in analogy with the disclosure of US Patent No. 4,867,748 or US patent No. 5,133,821 in order to reduce the risk of "rolling-up" the edge of the patch reducing the wear-time. The edge is preferably bevelled so that the thickness adjacent to the edge does not exceed about 30% of the maximum thickness of the patch; more preferred not exceeding 25% of the maximum thickness.

The patch of the invention has surprisingly good water resistance, and bevelling of the edge may further enhance these properties. Preferably the edges are bevelled to a thickness of approximately 50 my. By the expression "water resistance" is understood that after application of the patch to the body part, the patch is capable of resisting numerous times of exposures to water or humidity, such as bathing hand washing, swimming or perspiration.

The excellent water resistance and the good moisture handling qualities render it possible to achieve an extremely long wear time for the patch compared to commonly known products. By bevelling the edges of the patch to a very low thickness, or have an overall low thickness of the patch the wear time may be increased. The rolling-up of the edges of the patch during use may depend on the amount of exposed adhesive along the edge portion, so, the thinner layer of adhesive along the edge, the less rolling-up may occur.

The patch may be prepared by a one step process, where a high flexibility in production may be achieved, thin bevellings may be prepared, with adapted centre thickness, and at the same time sufficient capacity to absorb exudates from a wound or blister.

The adhesive of the patch of the invention may be any suitable skin-friendly adhesive. The adhesive further comprises particles of hydrocolloids and/or super absorbing particles or fibres.

The skin-friendly adhesive may be any skin-friendly adhesive known per se for production of medical articles, which are to be adhered to human skin, preferably an adhesive comprising hydrocolloids or other moisture absorbing constituents for prolonging the time of use. The adhesive may suitably be of the type disclosed in US patent Nos. 4,231,369, 4,367,732, 4,867,748, and 5,714,225. Especially preferred are the adhesives disclosed in US patent Nos. 4,367,732, and 5,714,225.

The patch of the present invention may in one embodiment of the invention be in the form of a mono-phase adhesive, i.e. made from one adhesive component or in accordance with another embodiment of the invention be in the form of a two-zone adhesive, e.g. of the general type disclosed in US patent No. 5,714,225, i.e. a part of or all of the adhesive areas of the patch having maximum thickness being constituted by more than one type of adhesive.

The particle size of the hydrocolloid particles influence on the thickness of the adhesive layer, as it is difficult to prepare an adhesive layer being thinner than the size of the hydrocolloid particles.

The physical form of conventional hydrocolloids is relative coarse and irregular particles, typically about 60-100 µm and the particles are in the form of a dry powder. In order to obtain finer particles, the hydrocolloid may be milled and/or sifted.

Suitable hydrocolloids for the patch of the present invention include synthetic polymers prepared from single or multiple monomers, naturally occurring hydrophilic polymers or chemically modified naturally occurring hydrophilic polymers. The hydrocolloid polymers may be linear or cross-linked. This include natural or chemically modified natural polymers like cellulosics such as CMC, chitosan, pectin, guar gum, starches or dextrines, collagenes and gelatine and synthetic polymers like polyacrylic acid, polyvinylealcohol/acetate, polyhydroxyalkyl acrylates and methacrylates, polyacrylamides, polystyrene sulfonates, polyvinyl pyrilidone, polyglycols, copolymers, grafts of such, copolymers or compositions of such.

In a preferred embodiment of the invention the hydrocolloid particles have an average size being substantially less than 125µm, more preferred less than 100 µm, even more preferred less than 75 µm and most preferred less than 50 µm.

The adhesive layer may be in the form of a pattern, such as geometric pattern or a random pattern, or the adhesive layer may comprise larger interruptions in the form of areas with no adhesive, e.g. the central part of the patch.

It is preferred that the adhesive layer is uninterrupted. The uninterrupted layer provides several advantages, such a less wrinkling, better invisibility and better blending into the skin. Backing layer being uncoated with adhesive may be more non-transparent and thus more visible. Furthermore, the pattern may leave marks on the skin when the patch is removed.

The adhesive patch may be in a flat continuous layer from 20 g/m² up to 1000 g_{/}m².

The patch of the invention is suitable for covering cuts, graces and abrasions, scars, wrinkles, discolouring of the skin or the like. The patch may be especially suitable for treatment of herpes, as the patch may be applied to the lip or lip region being discrete and without causing discomfort.

It is suitable that the backing layer is a substantially water-impervious film which protects the adhesive from being adversely affected when the wearer is bathing or in case of incidental wetting of the area and especially when the adhesive is water absorbing.

The backing layer may be any water impervious layer or film may be of any suitable material known per se for use in the preparation of wound dressings e.g. a foam, a non-woven layer or a polyurethane, polyethylene, polyester or polyamide film. In accordance with the invention it has been found in practice that the use of a thinner backing layer or film than is normally used when preparing medical dressings, an improved stretchability and adaptability is obtained at the same time as the modulus is reduced.

The backing layer may preferably be an elastic, flexible and non-sticking film that protects the adhesive during storage as well as during use.

The water impervious, but vapour permeable layer or film is preferably a low-friction flexible polymer film reducing the risk of unwanted stress in the area of application.

An especially suitable material for use as a water impervious film is a polyurethane film. A preferred low friction film material is disclosed in US patent No. 5,643,187.

The backing layer may have a suitable thickness for the intended use. If the patch were desired for an "invisible" face patch, a rather thin film would be appropriate. It is preferred that the backing layer has a thickness of less than 30 µm.

A preferred thickness of this film may be below 20 microns, more preferred about 12-18 microns, e.g. about 15 microns, thus resulting in a significant decrease of the modulus, compared to a film that is normally used when preparing medical dressings. An improved stretchability and adaptability is obtained at the same time as the modulus is reduced.

In accordance with a preferred embodiment the backing layer is a film showing a low surface friction. Furthermore, the surface may be opaque, with a reflection being near to the reflection of skin, thus enabling the patch to blend with the skin colour and reflection and be less visible.

The backing layer may be coloured in suitable colours, e.g. flesh-colour or it may carry ornamentals. The backing layer may be transparent, translucent, opaque or non-transparent, depending on the intended use.

In order to be able to visually blend into the skin and become invisible, it is desired that the patch have a reflectance being close to that of the skin. It is preferred that the reflectance is lower than 5, more preferred between 4,5 and 1, and even more preferred between 4 and 1,5. The reflectance is measured on a Micro-TRI-gloss apparatus from BYK-Gardner and is measured with reference to an ASTM D523 standard. The measuring angle is 60°. The higher value of reflectance, the higher is the gloss of the product.

A patch of the invention may preferably be sterilised for avoiding the risk of causing infections when applied to skin areas having broken skin.

It is not critical whether or not the patch is sterilised, if the patch is applied to non-broken skin, e.g. on a face portion for camouflaging irregularities of the skin, such as scars.

Preventing and protecting against abrasion may also be considered as an aspect of the present invention.

The patch of the invention is optionally covered in part or fully by one or more release liners or cover films to be removed before or during application.

A protective cover or release liner may for instance be siliconized paper. It does not need to have the same contour as the patch, e.g. a number of patches may be attached to a larger sheet of protective cover. The protective cover is not present during the use of the patch of the invention and is therefore not an essential part of the invention.

Furthermore, the patch of the invention may comprise one or more "non touch" grip(s) known per se for applying the patch to the skin without touching the adhesive layer. Such a non-touch grip is not present after application of the patch. For larger patches it is suitable to have 2 or 3 or even 4 "non-touch" grips.

The patch of the invention may further comprise one or more cover layers. The cover layer may protect the patch during storage and help easy application of the patch. The cover layer is removed during or after application.

Preferably, in all embodiments of the present invention, the patch is provided in the form of a backing layer with an adhesive applied to one surface thereof. The adhering surface of the patch may comprise a pharmaceutically active substance. For example, emollients or e.g. retinoids for treating or preventing formation of psoriasis, eczema, callous, skin, corns or blisters. Examples of applicable pharmaceutical medicaments include a cytochine, such as a growth hormone or a polypetide growth factor such as TGF, FGF, PDGF, EGF, IGF-1, IGF-2, colony stimulating factor, transforming growth factor, nerve stimulating growth factor and the like giving rise to the incorporation of such active substances in a form being apt to local application in a wound in which the medicament may exercise its effect on the wound, other medicaments such as bacteriostatic or bactericidal compounds, e.g. iodine, iodopovidone complexes, chloramine, chlorhexidine, silver salts such as sulphadizine, silver nitrate, silver acetate, silver lactate, silver sulphate, silver sodium thiosulphate or silver chloride, zind or salts thereof metronidazol, sulpha drugs, and pencillins, tissue-healing enhancing agents, e.g. RGD tripeptides and the like, proteins, amino acids such as taurine, vitamins such as ascorbic acid, enzymes for cleansing of wounds, e.g. pepsin, trypsin and the like, proteinase inhibitors for use in e.g. surgical insertion of the dressing in cancer tissue and/or other therapeutic agents which optionally be used for topical application, pain relieving agents such as NSAIDS, lidocaine or chinchocaine, emollients, retinoids or agents having a cooling effect.

Due to its discrete appearance and the easy applicability provided by the carrier system, the patch of the invention may advantageously be used for facial application, such as for the treatment of herpes, acne and warts with medicaments known per se for such purposes being contained in the adhesive or being applied thereto. Suitable anti viral medicaments for the treatment of herpes may for example comprise aciclovir or penciclovir. Azelain acid or isotretinoin may be used in a medicament for the treatment of acne. In respect of the treatment of warts, a mitotic inhibitor, such as podophyllotoxin, is applicable. Warts and/or callous skin may be treated by salicylic acid-based medicaments. Patches for treatment of acne, scratches or wounds may e.g. comprise antiseptic/antibiotic substances, vitamin compounds or other wound healing substances.

The above mentioned pharmaceutically active substances may be applied to the adhering surface of the dressing sheet after completion of the adhering coating, or they may be mixed into the adhesive prior to coating thereof onto the backing layer.

In one embodiment of the invention the medicament may be applied to the patch before application. An amount of a gel or cream may be applied to the central part of the patch before application to the treatment site. In the treatment of Herpes it may be advantageous to apply an Acyclovir containing cream or gel such a Zovir before application to the Herpes site.

The patch may comprise one or more cavities for accommodating a medicament. The cavities may be in the form of a dome shaped portion or an indentation. The patch may comprise an absorbent pad. The absorbent pad may be any suitable absorbent material, such as gauze, alginates, hydrocolloids, foam or super absorbers. In one embodiment of the invention the pad comprises an adhesive. The adhesive may be the same adhesive as on the patch or it may be a different adhesive, e.g. a more absorbent adhesive.

### EXAMPLES

### EXAMPLE 1

The reflectance of a patch according to the invention has been measured. The test has been carried out using a Micro-TRI-gloss apparatus from BYK-Gardner. The reflectance is measured with reference to an ASTM D523 standard. The angle was 60°. The higher value, the higher gloss of the product.

4 measurements on 3 persons were obtained, testing skin, a patch according to the present invention and a competitor product "T-zone". The T-zone patch is from Brodie & Stone plc and is a product for treatment of acne and comprises a top film coated with polyacrylate adhesive. The product further comprises an antiseptic agent in the form of Tea tree oil. The results of the reflectance test are shown in Table 1 below.

As can be seen from the results, the reflection of the patch of the present invention is very close to the reflection of the skin, and thus contributes to the invisibility of the patch. The competitor product, T-zone, has a much higher reflectance, and is thus more visible, and would not be suitable for a discrete appearance.

**TABLE 1**

| | **Skin** | **Patch** | **T-zone** |
|---|---|---|---|
| **Person 1** | | | |
| #1 | 2,6 | 2,8 | 21,4 |
| #2 | 2,6 | 3,1 | 18,2 |
| #3 | 2,6 | 2,9 | 26,6 |
| #4 | 3,2 | 2,3 | 16,9 |

| **Person 2** | | | |
|---|---|---|---|
| #1 | 2,1 | 2,7, | 10,0 |
| #2 | 1,8 | 3,6 | 6,2 |
| #3 | 2,3 | 2,2 | 6,0 |
| #4 | 2,4 | 3,3 | 5,0 |

| **Person 3** | | | |
|---|---|---|---|
| #1 | 2,7 | 4,2 | 10,3 |
| #2 | 2,8 | 4,4 | 9,6 |
| #3 | 2,7 | 3,2 | 21,2 |
| #4 | 3,0 | 3,5 | 20,7 |

### EXAMPLE 2

A clinical investigation has been carried out at Bispebjerg Hospital, Department of Dermato-venerology and Wound Healing Center. 85 persons with Herpes Labialis completed the study. The patients were provided with patches of the present invention during an outbreak of the virus and filled in a questionnaire. Results from the questionnaire are shown below.

94% answered that the patch was extremely or very elastic/flexible during the outbreak (followed the movements of the skin). 91 % answered that the patch was extremely pleasant to wear during the outbreak. 85% said that the patch relieved the outbreak. 88% said that the patch made the outbreak less visible. 81 % said that the cold sore did not form a crust, and among these found 86% that this was an advantage. 79% would be interested to buy the patches if they were obtainable from pharmacies/drug stores. 87% found that the patches made the outbreak easier to cope with / live with compared to traditional treatment. 65% found that the patches decreased the healing time of the outbreak.

It is concluded from the study that the patch of the present invention provides reduced healing time, and higher comfort during the outbreak, compared to traditional Herpes treatment.

### EXAMPLE 3

A comparison between treatment of Herpes Labialis with an Acyclovir containing cream and treatment with a patch of the present invention. The study was performed by Bispebjerg Hospital, Department of Dermato-venerology and wound Healing Center. The results are shown in Table 2.

**TABLE 2**

| | Treatment with cream | Treatment with patch |
|---|---|---|
| Redness/swollenness of skin in area of herpes | yes | yes |
| Blister formation | yes | only small blisters |
| Moist ulceration | yes | less ulceration |
| Formation of crust | yes | no |
| Secondary infection | maybe | no |

The treatment with the patch of the present invention moderates the formation of blisters and prevents the formation of a crust. The absence of crust seems to reduce the healing time and cause less inconvenience for the patient. Secondary infections are avoided, which also contributes to reduce healing time.

### EMBODIMENTS

1. A patch for covering a portion of the anatomical surface of a living being, said patch being able to adhere to the skin or mucosa, and/or a wound, said patch comprising a backing layer and a layer of a skin-friendly adhesive for adhering to the skin or mucosa, said adhesive comprises hydrocolloid particles, wherein at least along the periphery of the patch the thickness of the adhesive layer is 20-300 µm and the vapour permeability of the patch is 200-1000 g/m² and the absorption of the patch is 40-600 g/m²/6h.
2. A patch according to embodiment 1, wherein the patch has a substantially uniform thickness.
3. A patch according to any of the preceding embodiments wherein the absorption of the patch is 50-400 g/m²/6h.
4. A patch according to any of the preceding embodiments wherein the thickness of the adhesive layer is 30-200 µm.
5. A patch according any of the preceding embodiments wherein the vapour permeability of the patch is 300-800 g/m².
6. A patch according to any of the preceding embodiments wherein the hydrocolloid particles have a size being substantially less than 125 µm.
7. A patch according to any of the preceding embodiments wherein the hydrocolloid particles have a size being substantially less than 50 µm.
8. A patch according to any of the preceding embodiments, wherein the patch has a reflectance lower than 5.
9. A patch according to any of the preceding embodiments wherein the backing layer is a polyurethane film.
10. A patch according to any of the preceding embodiments wherein the backing layer has a thickness of less than 30 µm.
11. A patch according to any of the preceding embodiments wherein the adhesive layer is uninterrupted.
12. A patch according to any of the preceding embodiments wherein the patch comprises an absorbent pad.
13. A patch according to any of the preceding embodiments wherein the patch comprises one or more cavities.
14. A patch according to any of the preceding embodiments, wherein the patch further comprises one or more active ingredients.

## Claims

1. A patch for covering a portion of the anatomical surface of a living being, said patch being able to adhere to the skin or mucosa, and/or a wound, said patch comprising a backing layer and a layer of a skin-friendly adhesive for adhering to the skin or mucosa, said adhesive comprises hydrocolloid particles, wherein at least along the periphery of the patch the thickness of the adhesive layer is 20-300 µm, wherein the patch has a reflectance lower than 5.

2. A patch according to claim 1, wherein the reflectance is between 4,5 and 1.

3. A patch according to any of the preceding claims, wherein the patch has a substantially uniform thickness.

4. A patch according to any of the preceding claims, wherein the absorption of the patch is 50-400 g/m²/6h.

5. A patch according to any of the preceding claims, wherein the thickness of the adhesive layer is 30-200 µm.

6. A patch according to any of the preceding claims, wherein the surface area of the patch is less than 5 cm².

7. A patch according to any of the preceding claims, wherein the hydrocolloid particles have a size being substantially less than 125 µm.

8. A patch according to any of the preceding claims, wherein the hydrocolloid particles have a size being substantially less than 50 µm.

9. A patch according to any of the preceding claims, wherein the backing layer is a polyurethane film.

10. A patch according to any of the preceding claims, wherein the backing layer has a thickness of less than 30 µm.

11. A patch according to any of the preceding claims, wherein the patch further comprises one or more active ingredients.

12. A patch according to claim 12, wherein the active ingredient is mixed into the adhesive.

13. A patch according to claim 12, wherein the active ingredient is zinc or salts thereof.

14. A patch according to any of claims 12-13, wherein the active ingredient is an anti viral medicament.

15. A patch according to any of the claims 12-14, wherein the anti viral medicament comprises aciclovir or penciclovir.
